# EUROPEAN PATENT APPLICATION

(11) **EP 2 078 720 A1**
(43) Date of publication of application: **15.07.2009**
(21) Application number: 07291644.8
(22) Date of filing: 28.12.2007
(51) Int. Cl.: C07D 487/04, C07D 241/44, A61K 31/495, C09B 5/24

(54) **Novel method for the preparation of derivatives of dihydrotetraazapentacenes, products such as obtained and uses thereof**

(71) Applicant: Université de la Méditerranée - Aix-Marseille II, 13007 Marseille (FR); CENTRE NATIONAL DE LA RECHERCHE SCIENTIFIQUE (CNRS), 75016 Paris (FR)
(72) Inventor: Siri, Olivier, 13720 Belcodene (FR); Seillan, Claire, 13008 Marseille (FR)
(74) Representative: Colombet, Alain André

(57) **Abstract**

The present invention relates to a method for the preparation of compounds having the following formula (I): 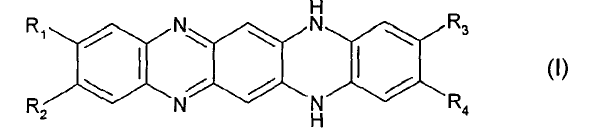 in which:
R₁, R₂, R₃, and R₄ represent, independently from each other, in particular H or an alkyl group having from 1 to 20 carbon atoms,
said method comprising the reaction of a compound of formula (II) 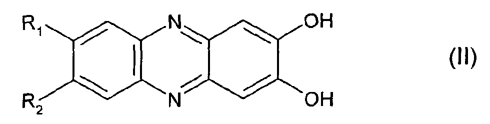 R₁ and R₂ being as defined above,
with a compound having the following formula (III): 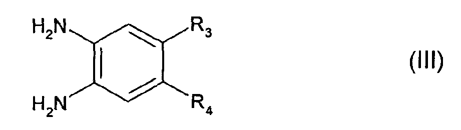 R₃ and R₄ being as defined above,
in the presence of a catalytic amount of an acid.

## Description

The present invention concerns a novel method for the preparation of derivatives of dihydrotetraazapentacenes, and derivatives of dihydrotetraazapentacenes as obtained by said method.

The present invention also concerns the use of said derivatives of dihydrotetraazapentacenes, in particular, as dyes, pigments, or chromophores.

Molecules exhibiting an extended conjugated pi system such as oligoacenes have attracted considerable interests for decades owing to their remarkable properties for electronic plastic and photonic devices (V. Coropceanu, J. Cornil, D. A. d. S. Filho, Y. Olivier, R. Silbey, J.-L. Brédas, Chem. Rev. 2007, 107, 926; A. R. Murphy, J. M. J. Fréchet, Chem. Rev. 2007, 107, 1066; J. G. C. Veinot, T. J. Marks, Acc. Chem. Res. 2005, 38, 632; and S. Günes, H. Neugebauer, N. S. Sariciftci, Chem. Rev. 2007, 107, 1324). Recent theoretical studies (M. Winkler, K. N. Houk, J. Am. Chem. Soc. 2007, 129, 1805) suggested the use of polyazaacenes since the successive replacement of CH moieties by nitrogen atoms in oligoacenes offers number of opportunity to manipulate and control the electronic properties, the stability and the crystal structure in the solid state. Such compounds are very attractive for electronic applications such as organic thin-film transistors (OTFTs)(Y. Ma, Y. Sun, Y. Liu, J. Gao, S. Chen, X. Sun, W. Qiu, G. Yu, G. Cui, W. Hu, D. Zhu, J. Mater. Chem. 2005, 15, 4894), organic light emitting diodes (OLEDs) (M. B. Casu, P. Imperia, S. Schrader, B. Falk, Surface Science 2001, 482-485, 1205; M. B. Casu, P. Imperia, S. Schrader, B. Falk, M. Jandke, P. Strohriegl, Synthetic Metals 2001, 124, 79) and photovoltaic cells (M. Antinucci, B. Chevalier, A. Ferriolo, Solar Energy Materials and Solar Cells 1995, 39, 271), owing to the presence of heterocyclic rings that are responsible for a high ionisation potential, high electron affinity, and a better stability.

Only few examples of substituted dihydrotetraazapentacenes (DHTAP) are reported in the literature. In particular, N- and C-substituted systems (O. Fischer, E. Hepp, Chem. Ber. 1890, 23, 1789; O. Fischer, E. Hepp, **1895,** *28*, 293; O. N. Witt, Chem. Ber. 1887, 20, 1538; M. A. Hassan, R. F. Fandy, T. M. EI-Amine, J. Heterocyclic Chem. 2001, 38, 179) were prepared but not used for electronic applications. In particular, WO2007/066098 describes a one-pot procedure which limits the control of the condensation owing to the presence of a mixture of four reagents and which requires the need of a large amount of acid, and WO 94/29314 discloses a two steps preparation in oxidizing medium (via the diaminophenazine intermediate) for which tuning the four terminal substituents is not possible.

An aim of the present invention is to provide a method for preparing derivatives of dihydrotetraazapentacenes, which is more versatile and more ecological than the prior art methods.

An aim of the present invention is also to provide a method for the preparation of a plurality of substituted derivatives, wherein the number and the nature of said substituents can be controlled.

An aim of the present invention is also to provide a method for preparing various derivatives of dihydrotetraazapentacenes, said derivatives containing a plurality of various substituents.

An aim of the invention is also to provide new derivatives of dihydrotetraazapentacenes, and in particular dissymmetrical derivatives.

The present invention relates to a method for the preparation of compounds having the following formula (I): 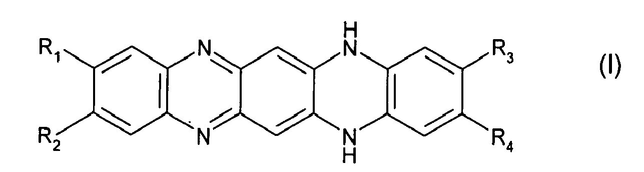 in which:
R₁, R₂, R₃, and R₄ represent, independently from each other, a substituent selected from the group consisting of:
   . H
   - alkyl group having from 1 to 20, and preferably from 1 to 10, carbon atoms,
   - alkenyl group having from 1 to 6 carbon atoms,
   - alkynyl group having from 1 to 6 carbon atoms,
   - aryl or heteroaryl group having 6 to 30, and preferably from 6 to 10, carbon atoms,
   - (C₆-C₁₀)aryl(C₁-C₄)alkyl group,
   - acyl group having from 1 to 20 carbon atoms, in particular CO-R group, wherein R is H, an alkyl group, an alkenyl group, an alkynyl group, or an aryl group, as defined above,
   - NR'R", wherein R' and R" represent, independently from each other, H, an alkyl group, an alkenyl group, an alkynyl group, or an aryl group, as defined above,
   - CONR'R", wherein R' and R" are as defined above,
   - NHCOR, wherein R is as defined above,
   - COOR, wherein R is as defined above,
   - OCOR, wherein R is as defined above,
   - OR, wherein R is as defined above,
   - SR, wherein R is as defined above,
   - SO₂R, wherein R is as defined above,
   - SO₂NR'R", wherein R' and R" are as defined above,
   - SO₃H,
   - halogen such as Cl, F, Br or I,
   - CF₃,
   - CN, and
   - nitro,
      or salts thereof,
      said method comprising the reaction of a compound of formula (II) 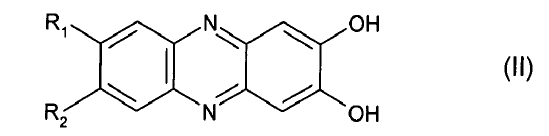 R₁ and R₂ being as defined above,
      with a compound having the following formula (III): 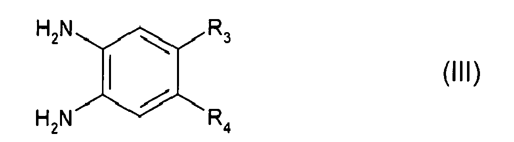 R₃ and R₄ being as defined above,

in the presence of a catalytic amount of an acid selected from the group consisting of: acetic acid, trifluoroacetic acid, and diluted hydrochloric acid.

The reaction of a compound of formula (II) with a compound of formula (III) is thus carried out without any solvent.

The above-mentioned catalytic amount of acid is preferably comprised from 0.1 mL to 5 mL, and in particular from 0.5 to 1 mL, for 1 mmol of compound of formula (II), and most preferably said catalytic amount is 1 mL for 1 mmol of compound of formula (II).

As used herein, the term "alkyl" refers to a hydrocarbon chain that may be a straight chain or branched chain, containing the indicated number of carbon atoms. For example, C₁-C₁₂ alkyl indicates that the group may have from 1 to 12 (inclusive) carbon atoms in it. Said alkyl groups can be methyl, ethyl, propyl, isopropyl, butyl, isobutyl, sec-butyl, tert-butyl, pentyl, isoamyl, neopentyl, 1-ethylpropyl, 3-methylpentyl, 2,2-dimethylbutyl, 2,3-dimethylbutyl, or hexyl.

Said alkyl group can also be a cycloalkyl group. The term "cycloalkyl" as employed herein includes saturated cyclic, bicyclic, tricyclic, or polycyclic hydrocarbon groups having 3 to 12 carbons, wherein any ring atom capable of substitution can be substituted by a substituent. Examples of cycloalkyl moieties include, but are not limited to, cyclohexyl and adamantyl.

The term "substituents" refers to a group "substituted" on an alkyl, alkenyl, alkynyl, cycloalkyl, aryl, or heteroaryl group at any atom of that group. Suitable substituents include, without limitation, alkyl, alkenyl, alkynyl, alkoxy, halo, hydroxy, cyano, nitro, amino, SO₃H, sulfate, phosphate, perfluoroalkyl, perfluoroalkoxy, methylenedioxy, ethylenedioxy, carboxyl, oxo, thioxo, imino (alkyl, aryl, aralkyl), S(O)ₙ alkyl (where n is 0-2), S(O)ₙ aryl (where n is 0-2), S(O)ₙ heteroaryl (where n is 0-2), S(O)ₙ heterocyclyl (where n is 0- 2), amine (mono-, di-, alkyl, cycloalkyl, aralkyl, heteroaralkyl, and combinations thereof), ester (alkyl, aralkyl, heteroaralkyl), amide (mono-, di-, alkyl, aralkyl, heteroaralkyl, and combinations thereof), sulfonamide (mono-, di-, alkyl, aralkyl, heteroaralkyl, and combinations thereof), unsubstituted aryl, unsubstituted heteroaryl, unsubstituted heterocyclyl, and unsubstituted cycloalkyl.

As used herein, the term "alkenyl" refers to a nonaromatic hydrocarbon chain that may be a straight chain or branched chain, containing at least one carbon-carbon double bond, and having the indicated number of carbon atoms. This term also includes the cycloalkenyl and heterocycloalkenyl groups.

The term "cycloalkenyl" as employed herein includes partially unsaturated, nonaromatic, cyclic, bicyclic, tricyclic, or polycyclic hydrocarbon groups having 5 to 12 carbons, preferably 5 to 8 carbons, wherein any ring atom capable of substitution can be substituted by a substituent. Examples of cycloalkyl moieties include, but are not limited to cyclohexenyl, cyclohexadienyl, or norbornenyl.

The term "heterocycloalkenyl" refers to a partially saturated, nonaromatic 5- 10 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S (e.g., carbon atoms and 1-3, 1-6, or 1-9 heteroatoms of N, O, or S if monocyclic, bicyclic, or tricyclic, respectively), wherein any ring atom capable of substitution can be substituted by a substituent.

As used herein, the term "alkynyl" refers to a nonaromatic hydrocarbon chain that may be a straight chain or branched chain, containing at least one carbon-carbon triple bond, and having the indicated number of carbon atoms.

The term "acyl" refers to an alkylcarbonyl, cycloalkylcarbonyl, arylcarbonyl, heterocyclylcarbonyl, or heteroarylcarbonyl substituent, any of which may be further substituted by substituents.

The term "aryl" refers to an aromatic monocyclic, bicyclic, or tricyclic hydrocarbon ring system, wherein any ring atom capable of substitution can be substituted by a substituent. Examples of aryl moieties include, but are not limited to, phenyl, naphthyl, and anthracenyl.

As used herein, the term "arylalkyl" refers to an alkyl group that is substituted with an aryl group, wherein the alkyl and aryl groups are as defined above. Examples of arylalkyl groups include, but are not limited to, benzyl, bromobenzyl, phenethyl, benzhydryl, diphenylmethyl, triphenylmethyl, diphenylethyl, naphthylmethyl, and 9-fluorenyl groups etc.

The term "heterocyclyl" refers to a nonaromatic 3-10 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S (e. g. , carbon atoms and 1-3, 1-6, or1-9 heteroatoms of N, O, or S if monocyclic, bicyclic, or tricyclic, respectively), wherein any ring atom capable of substitution can be substituted by a substituent.

The term "heteroaryl" refers to an aromatic 5-8 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S (e. g. , carbon atoms and 1-3, 1-6, or 1-9 heteroatoms of N, O, or S if monocyclic, bicyclic, or tricyclic, respectively), wherein any ring atom capable of substitution can be substituted by a substituent.

According to a preferred embodiment of the method of the present invention, the compound of formula (II) as mentioned above is obtained by the reaction of 2,5-dihydroxy-1,4-benzoquinone with a compound having the following formula (IV): 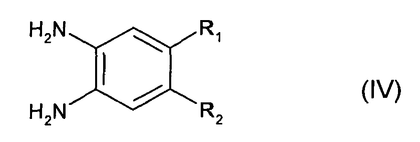 R₁ and R₂ being as defined above,
said reaction being carried out in water or ethanol, or mixtures thereof.

This step is carried out in the presence of non toxic solvents.

The present invention relates to a method for the preparation of compounds having the following formula (I): 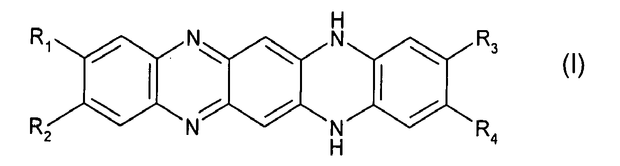 in which:
R₁, R₂, R₃, and R₄ represent, independently from each other, a substituent selected from the group consisting of:
- H
- alkyl group having from 1 to 20, and preferably from 1 to 10, carbon atoms,
- alkenyl group having from 1 to 6 carbon atoms,
- alkynyl group having from 1 to 6 carbon atoms,
- aryl or heteroaryl group having 6 to 30, and preferably from 6 to 10, carbon atoms,
- (C₆-C₁₀)aryl(C₁-C₄)alkyl group,
- acyl group having from 1 to 20 carbon atoms, in particular CO-R group, wherein R is H, an alkyl group, an alkenyl group, an alkynyl group, or an aryl group, as defined above,
- NR'R", wherein R' and R" represent, independently from each other, H, an alkyl group, an alkenyl group, an alkynyl group, or an aryl group, as defined above,
- CONR'R", wherein R' and R" are as defined above,
- NHCOR, wherein R is as defined above,
- COOR, wherein R is as defined above,
- OCOR, wherein R is as defined above,
- OR, wherein R is as defined above,
- SR, wherein R is as defined above,
- SO₂R, wherein R is as defined above,
- SO₂NR'R", wherein R' and R" are as defined above,
- SO₃H,
- halogen such as Cl, F, Br or I,
- CF₃,
- CN, and
- nitro,
   or salts thereof,
   said method comprising:
   a) the reaction of 2,5-dihydroxy-1,4-benzoquinone with a compound having the following formula (IV): 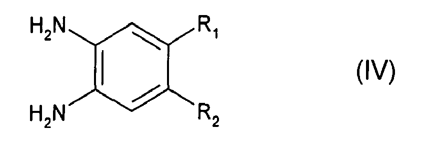 R₁ and R₂ being as defined above,
      said reaction being carried out in water or ethanol, or mixtures thereof,
      to obtain a compound of formula (II) as follows: 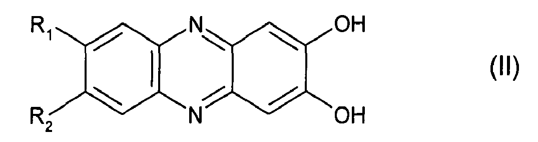 R₁ and R₂ being as defined above,
      said compound of formula (II) being then isolated,
   b) and the reaction of said compound of formula (II) with a compound having the following formula (III): 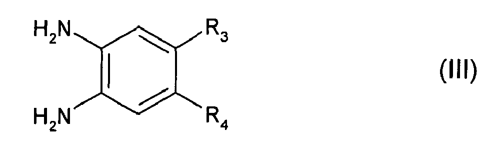 R₃ and R₄ being as defined above,
      in the presence of a catalytic amount of an acid selected from the group consisting of: acetic acid, trifluoroacetic acid, and diluted hydrochloric acid, to obtain said compound of formula (I).
   The present invention also relates to compounds having the following
   formula (I): 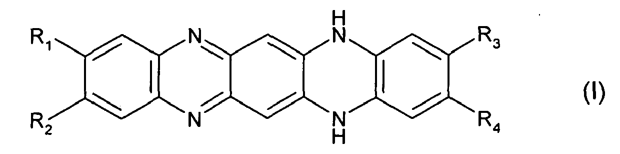 in which:
   R₁, R₂, R₃, and R₄ represent, independently from each other, a substituent selected from the group consisting of:
   . H
   - alkyl group having from 1 to 20, and preferably from 1 to 10, carbon atoms,
   - alkenyl group having from 1 to 6 carbon atoms,
   - alkynyl group having from 1 to 6 carbon atoms,
   - aryl or heteroaryl group having 6 to 30, and preferably from 6 to 10, carbon atoms,
   - (C₆-C₁₀)aryl(C₁-C₄)alkyl group,
   - acyl group having from 1 to 20 carbon atoms, in particular CO-R group, wherein R is H, an alkyl group, an alkenyl group, an alkynyl group, or an aryl group, as defined above,
   - NR'R", wherein R' and R" represent, independently from each other, H, an alkyl group, an alkenyl group, an alkynyl group, or an aryl group, as defined above,
   - CONR'R", wherein R' and R" are as defined above,
   - NHCOR, wherein R is as defined above,
   - COOR, wherein R is as defined above,
   - OCOR, wherein R is as defined above,
   - OR, wherein R is as defined above,
   - SR, wherein R is as defined above,
   - SO₂R, wherein R is as defined above,
   - SO₂NR'R", wherein R' and R" are as defined above,
   - SO₃H,
   - halogen such as Cl, F, Br or I,
   - CF₃,
   - CN, and
   - nitro,
      or salts thereof.

The compounds of the invention also include protonated forms of the compounds of formula (I) as mentioned above, which have in particular the following formula: 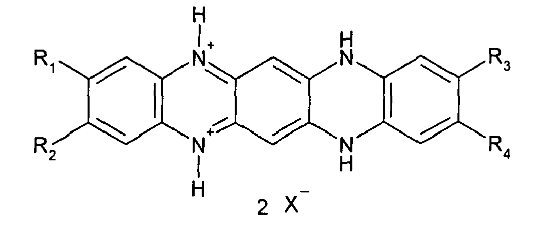 wherein R₁, R₂, R₃, and R₄ are as defined above, and wherein X is selected from the group consisting of: F₃COO, Cl, F, Br, I, BF₄, and H₃COO.

According to a preferred embodiment, at least one of the R₁, R₂, R₃, and R₄ groups of formula (I) is other than H.

Preferably, in formula (I), when one of the R₁, R₂, R₃, and R₄ groups is an acid group COOH or SO₃H, then none of the remaining R₁, R₂, R₃, and R₄ groups is an amide group such as CONH₂, CONR'R", SO₂NH₂ or SO₂NR'R", R' and R" being as defined above.

Preferably, in the compounds of the present invention, when R₁ = R₂ = R₃ = H, then R₄ is not COOH.

According to a particular embodiment, the compounds of the invention have the formula (I), wherein R₁ and R₂ are H.

According to another particular embodiment, the compounds of the invention have the formula (I), wherein R₁, R₂ and R₃ are H.

More particularly, the present invention relates to a compound of formula (I) wherein R₁, R₂ and R₃ are H, and R₄ is a halogen atom, in particular Cl.
When R₄ is Cl, the compound has the following formula:

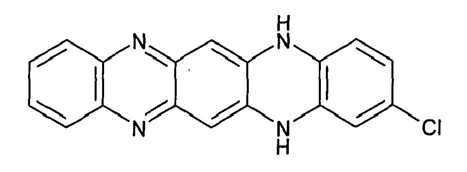

More particularly, the present invention also relates to a compound of formula (I) wherein R₁ and R₂ are H, and R₃ and R₄ are halogen atoms, in particular Cl.
When R₃ and R₄ are Cl, the compound has the following formula:

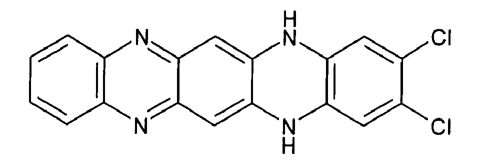

More particularly, the present invention also relates to a compound of formula (I) wherein R₁, R₂ and R₃ are H, and R₄ is COOR, R being as defined above, and being preferably H.
When R₄ is COOH, the compound has the following formula:

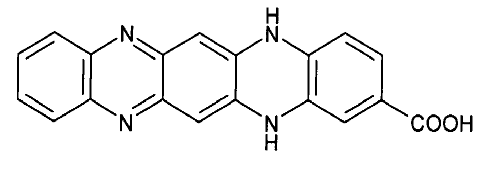

More particularly, the present invention also relates to a compound of formula (I) wherein R₁, R₂ and R₃ are H, and R₄ is an alkyl group, and in particular a methyl group.

More particularly, the present invention also relates to a compound of formula (I) wherein R₁, R₃ and R₄ are H, and R₂ is an alkyl group, and in particular a methyl group.
When R₂ is methyl, the compound has the following formula:

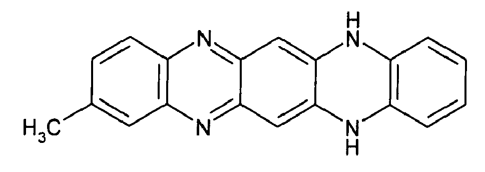

According to a particular embodiment, the compounds of the invention have the formula (I), wherein R₁ and R₂ are alkyl groups such as defined above, and in particular methyl groups.

According to another particular embodiment, the compounds of the invention have the formula (I), wherein R₁ and R₂ are alkyl groups, and R₃ is H.

More particularly, the present invention relates to a compound of formula (I) wherein R₁ and R₂ are alkyl groups, and R₃ and R₄ are H.
When R₁ and R₂ are methyl, the compound has the following formula:

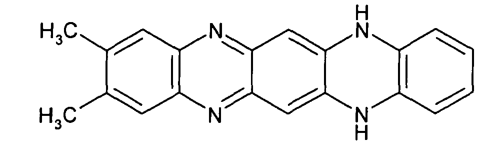

More particularly, the present invention also relates to a compound of formula (I) wherein R₁ and R₂ are alkyl groups, and R₃ is H, and R₄ is COOR, R being as defined above.
When R₁ and R₂ are methyl, and R is H, the compound has the following formula:

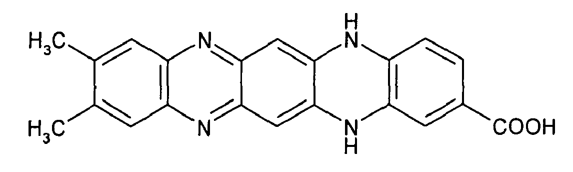

According to another particular embodiment, the compounds of the invention have the formula (I), wherein R₄ is COOR, R being as defined above, and being preferably H.

According to another particular embodiment, the compounds of the invention have the formula (I), wherein R₄ is a halogen atom, in particular Cl.

Other preferred compounds of the invention are compounds of formula (I) wherein R₁ and R₂ are OR, R being as defined above, and being preferably an alkyl group. Among these preferred compounds, the following is particularly preferred:

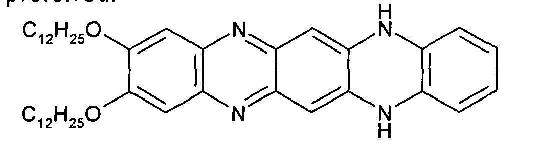

Other preferred compounds of the invention are compounds of formula (I), wherein R₁ and R₂ are OR, R being as defined above, and being preferably an alkyl group, and wherein R₄ is COOR. Among these preferred compounds, the following is particularly preferred:

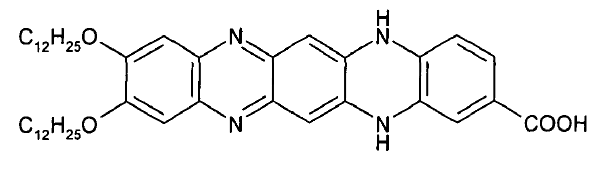

Other preferred compounds of the invention are compounds of formula (I), wherein R₁, R₂, R₃ and R₄ are OR, R being as defined above, and being preferably an alkyl group. Among these preferred compounds, the following is particularly preferred:

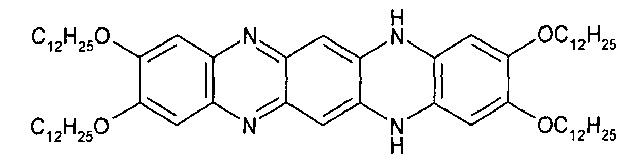

The present invention also relates to the intermediate compounds having the following formula (II): 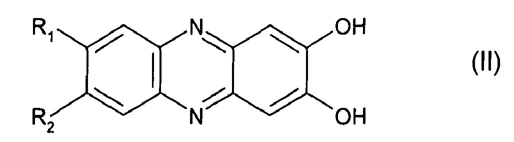 in which:
R₁, and R₂ represent, independently from each other, a substituent selected from the group consisting of:
. H
- alkyl group having from 1 to 20, and preferably from 1 to 10, carbon atoms,
- alkenyl group having from 1 to 6 carbon atoms,
- alkynyl group having from 1 to 6 carbon atoms,
- aryl or heteroaryl group having 6 to 30, and preferably from 6 to 10, carbon atoms,
- (C₆-C₁₀)aryl(C₁-C₄)alkyl group,
- acyl group having from 1 to 20 carbon atoms, in particular CO-R group, wherein R is H, an alkyl group, an alkenyl group, an alkynyl group, or an aryl group, as defined above,
- NR'R", wherein R' and R" represent, independently from each other, H, an alkyl group, an alkenyl group, an alkynyl group, or an aryl group, as defined above,
- CONR'R", wherein R' and R" are as defined above,
- NHCOR, wherein R is as defined above,
- COOR, wherein R is as defined above,
- OCOR, wherein R is as defined above,
- OR, wherein R is as defined above,
- SR, wherein R is as defined above,
- SO₂R, wherein R is as defined above,
- SO₂NR'R", wherein R' and R" are as defined above,
- SO₃H,
- halogen such as Cl, F, Br or I,
- CF₃,
- CN, and
- nitro,
   with the proviso that when R₁ is H, then R₂ is not H, and that when R₂ is
   H, then R₁ is not H.

Thus the compound having the formula (II) wherein R₁ = R₂ = H is excluded from the present invention.

The preferred intermediate compounds are chosen among the following:

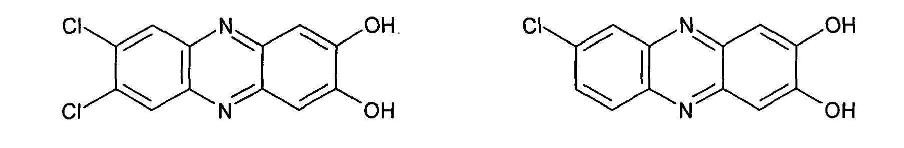

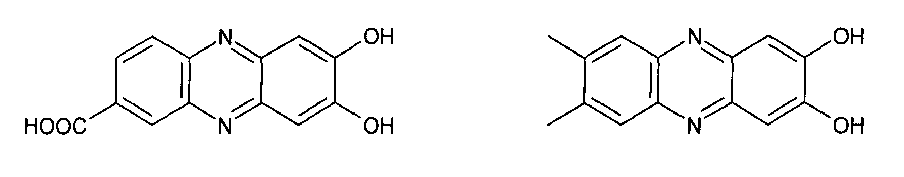

The present invention also relates to the use of the above-mentioned compounds of formula (I), as a chromophore or as a semi-conductive material.

More particularly, the non-protonated compounds are preferably used as chromophores, and the protonated compounds are preferably used as semi-conductive materials.

The compounds of the invention can be used as electron donors.

The present invention also relates to the use of a compound of formula (I) as mentioned above, wherein at least one of R₁, R₂, R₃, and R₄ groups is COOH, as a light-emitting molecule.

The present invention also relates to the use of a compound of formula (I) as mentioned above, as a dye or pigment.

The present invention also relates to a cosmetic composition comprising a compound of formula (I) as mentioned above, in association with a cosmetically acceptable vehicle.

The expression "cosmetically acceptable vehicle" refers to a vehicle adapted for a use in contact with animal and human cells, in particular epidermal cells, without any toxicity, irritation, nor undue and similar allergic response.

The present invention also relates to method for the cosmetic treatment, comprising the administration of a compound of formula (I) as mentioned above, or of the cosmetic composition as mentioned above.

### EXAMPLES

The commercially available 2,5-dihydroxy-*p*-benzoquinone reacted smoothly with various substituted o-diaminobenzenes (compounds of formula (IV)) **7-11** (1.1 equiv) in water (or alcohol) at 80°C under air to afford high yields of new substituted dihydroxyphenazines **13-16** (intermediate compounds of formula (II)) which were fully characterized (Scheme 1).

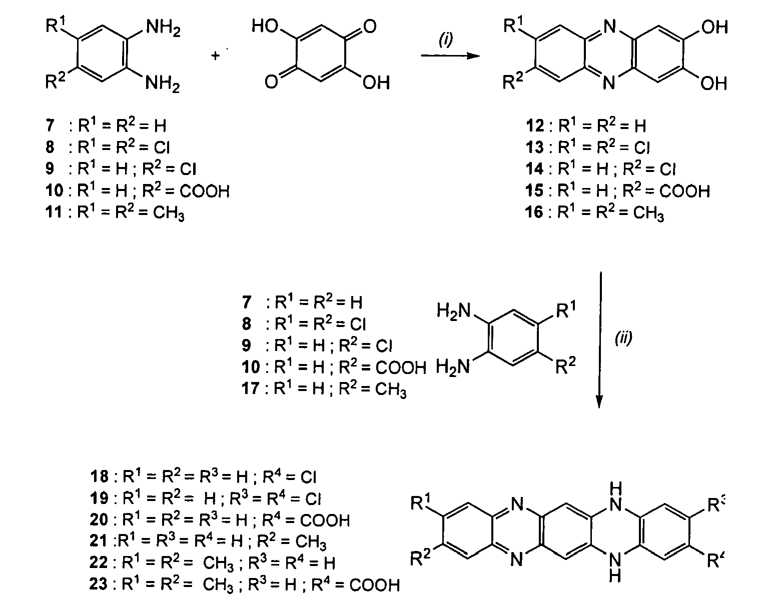

### Scheme 1. Synthesis of DHTAP 18-23: (i) H₂O or EtOH, 80°C, 24h. (ii) : catalytic amount of AcOH, 100°C, 24h.

The phenazine intermediates **12-16** are then heated in presence of a catalytic amount (few drops) of glacial acetic acid and a large excess of N-substituted o-diaminobenzenes (compounds of formula (III)) (5 - 10 equiv) for 24 hrs (solvent free synthesis) affording substituted DHTAP derivatives **18-23** (compounds of formula (I)) as dark purple solids.

### DETAILED PREPARATION OF THE COMPOUNDS OF THE INVENTION

### I - PREPARATION OF 2,3-DIHYDROXY-PHENAZINES

**Method for the preparation of analogues of 2,3-dihydroxy-phenazine**

A mixture of o-diaminobenzene (or analogue) (1 eq.) and of 2,5-dihydroxy-*p*-benzoquinone (1.1 to 2 éq.) in water or absolute ethanol is heated to reflux (1h30 to 24h). The end of the reaction is controlled by TLC (eluent: ethyl acetate). The reaction mixture is concentrated under reduced pressure. The crude residue is taken up in an organic solvent and the insoluble part is filtered on sintered glass. The number of equivalents of 2,5-dihydroxy-*p*-benzoquinone, the volume of ethanol, the reaction time and the precipitation solvent are indicated below for each compound.

The compounds are further characterized with ¹H and ¹³C NMR, MS and EA.

### 1- Synthesis of 2,3-dihydroxy-phenazine:

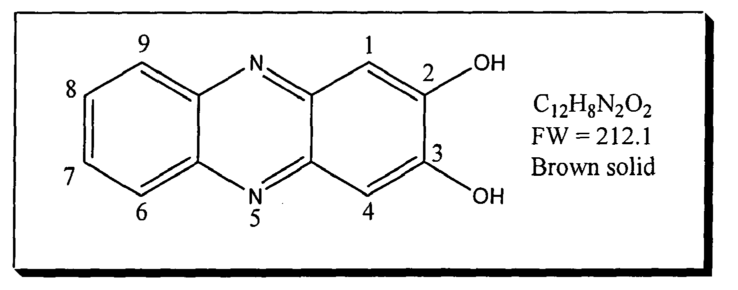

### Protocol 1:

The reaction involves 500 mg (4.62 mmol; 1 eq) of o-diaminobenzene, 1.3 g (9.32 mmol, 2 eq) of 2,5-dihydroxy-*p*-benzoquinone in 500 mL of ethanol. The mixture is heated to reflux for 1h30. The crude residue is then taken up in 20 mL of THF.

The desired compound is obtained as a brown solid and is obtained quantitatively.

### Protocol 2:

The reaction involves 250 mg (2.31 mmol; 1 eq) of o-diaminobenzene, 356.25 mg (2.54 mmol; 1.1 eq) of 2,5-dihydroxy-*p*-benzoquinone in 250 mL of ethanol. The mixture is heated to reflux for 24h. The crude residue is taken up in 10 mL of THF.

The desired compound is obtained as a brown solid, with a yield of 93%.

**¹H NMR** (DMSO *d₆*, ppm): 7.19 (s; 2H; H(1) and H(4)); 7.70 (dd; 2H; *J₁* = 6.5 *Hz*, *J₂* = 3.5 *Hz*; H(7) and H(8)); 8.02 (dd; 2H; *J₁* = 6.5 Hz, *J₂* = 3.5 Hz; H(6) and H(9)).
**¹³C NMR** (DMSO *d₆*, ppm): 106.49 (CH(1) and CH(4)); 127.56 (CH(6) and CH(9)); 128.31 (CH(7) and CH(8)); 141.55 (4 C^{IV}-**N**); 181.27 (C(2) and C(3)).
**MS:** [M-H⁺] = 211
**EA:** C = 67.16; H = 3.87; N = 13.04 (C₁₂H₈N₂O₂ . 1/9 EtOH: C = 67.55; H = 4.02; N = 12.89)

### 2- Synthesis of 2,3-dihydroxy-7,8-dichlorophenazine:

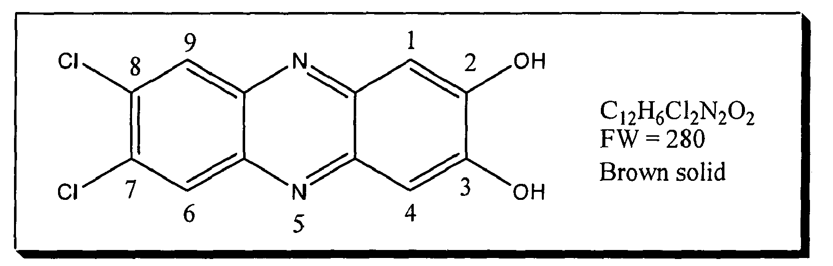

The reaction involves 300.7 mg (1.70 mmol; 1 eq.) of 1,2-diamino-4,5-dichlorobenzene, 262 mg (1.87 mmol; 1.1 eq.) of 2,5-dihydroxy-*p-*benzoquinone in 200 mL of ethanol. The mixture is then heated to reflux for 24h. The crude residue is taken up with a few mL of methanol. The insoluble part is rinsed with THF.

The desired compound is a brown solid and is obtained with a yield of 89%.

**¹H NMR** (DMSO *d₆*, ppm): 7.23 (s; 2H; H (1) and H(4)); 8.30 (s; 2H; H(6) and H(9)); 11.30 (se; 2 OH).
**MS:** [M-H]⁺ = 279
**AE:** Obtained values: C=50.8; H=2.3; N=10.3 (Theoretical values: C=51.27; H=2.15; N=9.97).

### 3- Synthesis of 2,3-dihydroxy-7-chlorophenazine:

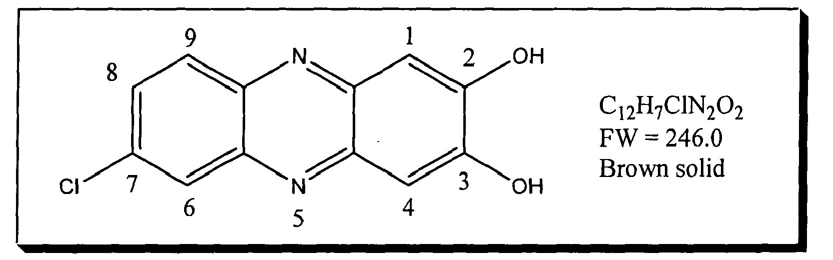

The reaction is carried out with 200.7 mg (1.41 mmol; 1 eq.) of 1,2-diamino-4-chloro benzene and 216.6 mg (1.55 mmol; 1.1 eq.) of 2,5-dihydroxy-*p*-benzoquinone in 160 mL of ethanol. The mixture is then heated to reflux for 24h. The crude residue is taken up in a few mL (5-10 mL) of ethanol.

The desired compound is a brown solid and is obtained with a yield of 90%.

**¹H NMR** (DMSO *d₆*, ppm): 7.26 (se; 2H; H(1) and H(2)); 7.73 (dd; 1H; *Jₒᵣₜₕₒ* = 9.25 *Hz; Jₘₑₜₐ* = 2.25 *Hz*; H(8)); 8.07 (d; 1 H; *Jₒᵣₜₕₒ* = 9.25 *Hz*; H(9)); 8.11 (se; 1H; H(6)); 11.13 (se; 2H; 2 OH).
**MS:** [M+H]⁺ = 247.7

### 4- Synthesis of 2,3-dihydroxy-phenazine-7-carboxylic acid:

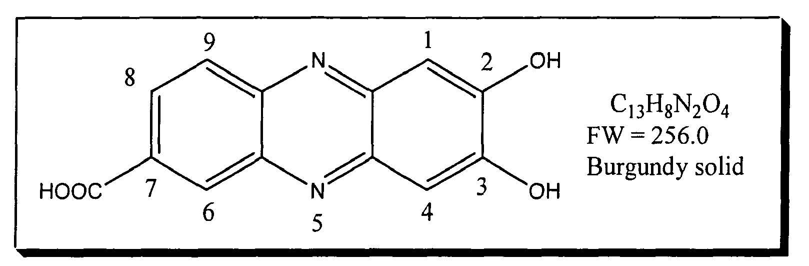

The reaction involves 500 mg (3.28 mmol; 1 eq) of 1,2-diamino-4-acido benzene, 500 mg (3.57 mmol; 1.1 eq) of 2,5-dihydroxy-*p*-benzoquinone in 300 mL of ethanol. The mixture is then heated to reflux for 23h. The crude residue is taken up in 20 mL of THF.

The desired compound is a dark burgundy solid and is obtained with a yield of 77%.

**¹H NMR** (DMSO *d₆*, ppm): 11,2 (se; 1H; COOH); 8.59 (s; 1H; H(6)); 8.16-8.13 (m; 2H; H(8) and H(9)); 7.28 (se; 2H; H(1) and H(4)).
**MS:** [M+H]⁺ m/z = 257; [M+Na]⁺ m/z =279
**AE :** C=54.97; H=3.70; N=9.42 (C₁₃H₈N₂O₄.3/2 H₂O: C=55.13; H=3.91; N=9.89).

### 5- Synthesis of 7,8-dimethyl-2,3-dihydroxy-phenazine:

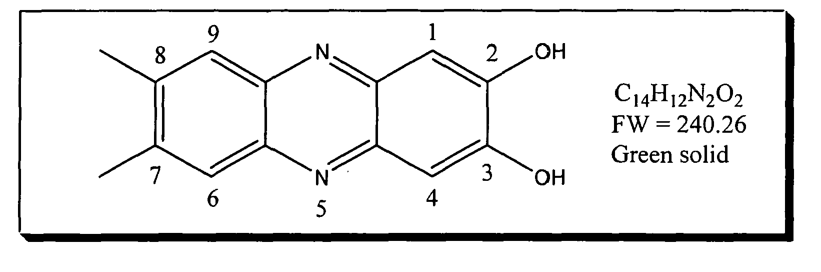

The reaction is carried out with 175 mg (1.28 mmol; 1 eq) of 1,2-diamino-4,5-dimethyl benzene and 198 mg (1.41 mmol; 1.1 eq) of 2,5-dihydroxy-p-benzoquinone in 140 mL of ethanol. The mixture is heated to reflux for 24h. The crude residue is taken up in 7 mL of THF. The desired compound is a dark green solid and is obtained with a yield of 63%.
**¹H RMN** (DMSO *d₆*, ppm): 10.80 (se; 2H; 2 OH) ; 7.79 (s; 2H; H(6) and H(9)); 7.22 (s; 2H; H(1) and H(4)); 2.46 (s; 6H; 2 CH3).
**SM:** [M+H]⁺ m/z = 241; [M+Na]⁺ m/z =263.
**AE:** C=66.13; H=5.86; N=10.00 (C₁₄H₁₂N₂O₂. 5/4 EtOH : C=66.54; H=6.60; N=9.41).

### II- PREPARATION OF DISSYMMETRICAL TETRAAZAPENTACENES

***General method for the preparation of dissymmetrical analogues of tetraazapentacene***

In a mortar, 1 eq of the intermediate 2,3-dihydroxy phenazine and 10 eq of an analogue of o-diaminobenzene are ground. The powder is transferred in a tube and glacial acetic acid is added (0.1 mL/0.1 mmol of the intermediate compound). The paste is heated at 100°C for 24h.

The volume of acetic acid, the conditions for the purification, the yield of the reaction as well as the details of the analyses are indicated below for each prepared compound.

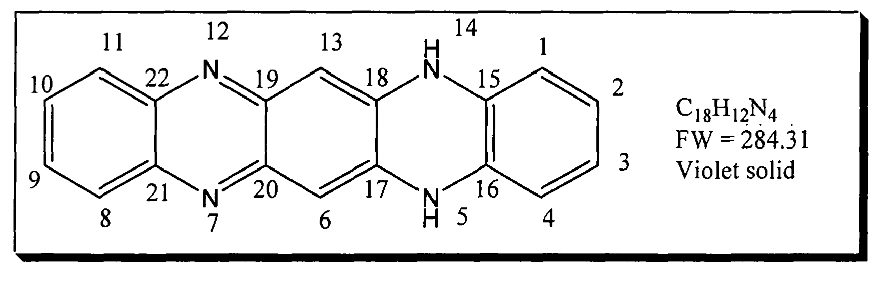

The reaction involves 831 mg (3.92 mmol; 1 eq) of 2,3-dihydroxy phenazine, 4.23 g (39.16 mmol; 10 eq) of o-diaminobenzene and 4 mL of glacial acetic acid. The reaction residue is taken up in dichloromethane, filtered on sintered glass, rinsed with dichloromethane and then with methanol until obtaining colourless mother liquors. The desired compound is a violet solid and is obtained with a yield of 83%.

**¹H NMR** (DMSO *d₆*, ppm): 9.70 (s; 2H; 2 NH); 7.68-7.40 (m; 4H; CH (8), (9), (10) and (11)); 6.62-6.47 (m; 4H; CH(1), (2), (3) and (4)); 6.32 (s; 2H; CH (6) and (13)).
**¹H,¹³C NMR CPMAS** (10 kHz): 147.1 and 145.7 (C (21) et (22)); 140.4 (C (18), (17), (20) and (19)); 129.7 (C (15) and (16)); 126.1 (CH (8), (9), (10) and (11)); 124.2; 122.8 (CH (3)); 120.7 (CH (2)); 114.5 (CH (1) and (4)); 99.5 and 99.0 (CH (6) and (13)).
**¹H,¹³C NMR CPPI** (C,CH₃): 147.1; 145.7; 140.4 and 129.7 (C).
**MS:** [M+H]+ = 285
**EA:** C = 74.21; N = 19.03; H = 4.13; C₁₈H₁₂N₄.1/3: C = 74.47; H = 4.4; N = 19.30).

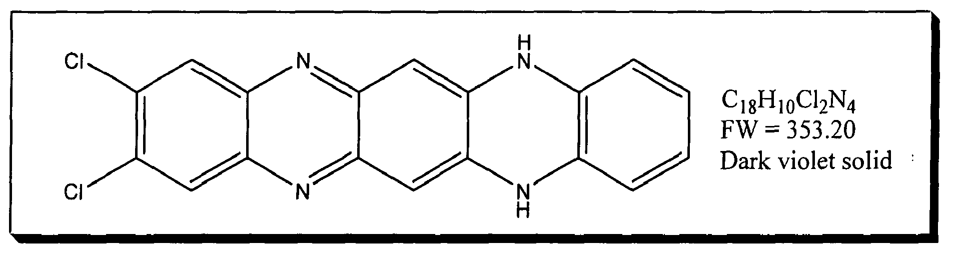

The reaction involves 200.6 mg (0.714 mmol; 1 eq) of 7,8-dichloro-2,3-dihydroxy phenazine, 771.5 mg (7.14 mmol; 10 eq) of o-diaminobenzene and 0.7 mL of glacial acetic acid. The reaction residue is taken up in dichloromethane, filtered on sintered glass, rinsed with dichloromethane and then with methanol until obtaining colourless mother liquors. The desired compound is a violet solid and is obtained with a yield of 72%.

**EA:** C = 59.5; H = 2.7; N = 15.6; C₁₈H₁₀Cl₂N₄. ½ H₂O: C = 59.69 ; H = 3.06; N = 15.47.

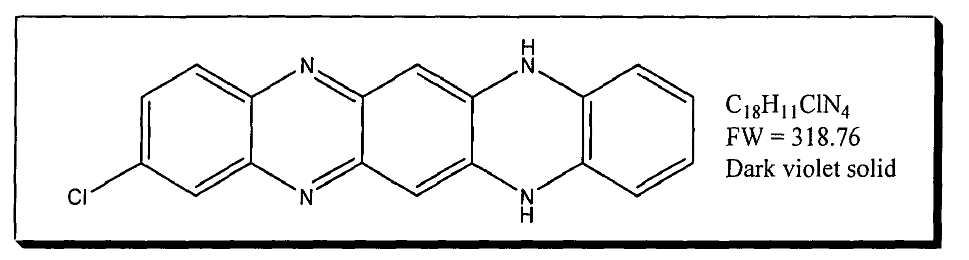

The reaction involves 150 mg (0.608 mmol; 1 eq) of 7-chloro-2,3-dihydroxy phenazine, 659.6 mg (6.10 mmol; 10 eq) of *o*-diaminobenzene and 0.6 mL of glacial acetic acid. The reaction residue is taken up in dichloromethane, filtered on sintered glass, rinsed with dichloromethane and then with methanol until obtaining colourless mother liquors. The desired compound is a violet solid and is obtained with a yield of 51%.
**MS:** [M+H]⁺ = 319.2; [M-H]⁺ = 317.2
**EA:** C = 65.3; H = 3.5; N = 16.6; C₁₈H₁₁ClN₄. ½ H₂O: C = 65.96 ; H = 3.69; N = 17.09.

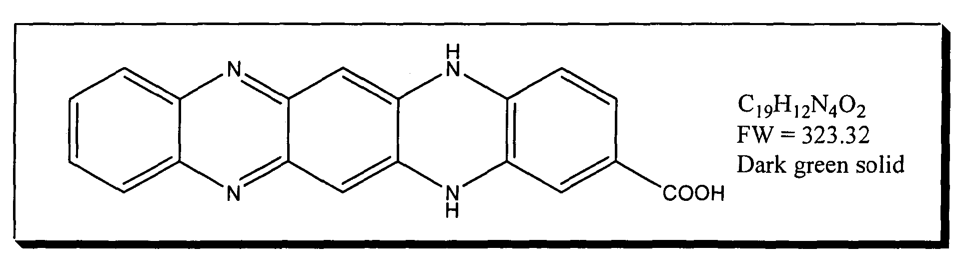

The reaction involves 201 mg (0.785 mmol; 1 eq) of 7-carboxy-2,3-dihydroxy phenazine, 848.4 mg (7.845 mmol; 10 eq) of *o*-diaminobenzene and 0.8 mL of glacial acetic acid. The reaction residue is taken up in dichloromethane, filtered on sintered glass, rinsed with dichloromethane and then with methanol until obtaining colourless mother liquors. The desired compound is a dark green solid and is obtained with a yield of 79%.
**¹H NMR** (DMSO *d₆*, ppm): 10.08 (s; 1 H; NH (5)); 9.85 (s; 1H; NH (14)); 7.72-7.45 (m; 4H; H (8), (9), (10) and (11)); 7.21 (d; 1H; J = 8.25 Hz; H(2)); 7.04 (s; 1 H; H(4)); 6.51 (d; 1 H; J = 8.25 Hz; H(1)); 6.44 (s; 1 H; H(6)); 6.36 (s; 1 H; H(13)).

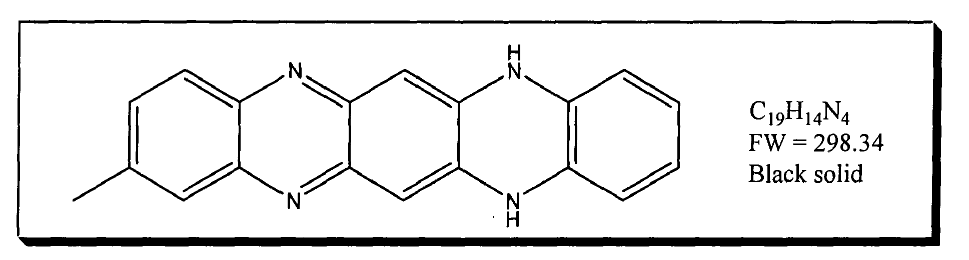

The reaction involves 200 mg (0.943 mmol; 1 eq) of 2,3-dihydroxy phenazine, 1.15 g (9.425 mmol; 10 eq) of 4-methyl-1,2diaminobenzene and 1 mL of glacial acetic acid. The reaction residue is taken up in dichloromethane, filtered on sintered glass, rinsed with dichloromethane and then with methanol until obtaining colourless mother liquors. The desired compound is a black solid and is obtained with a yield of 69%.
**¹H,¹³C NMR CPMAS** (10 kHz): 146.0 (C (21) and (22)); 140.3 (C (17), (18), (19) and (20)); 135.5 (C (9)); 130.0 (C (15) et (16)); 126.2 (CH (8), (10) et (11)); 120.9 (CH (2) and (3)); 114.4 (CH (3) and (4)); 99.8 (CH (6) and (13)); 22.8 (CH3).
**¹H,¹³C NMR CPPI** (C, CH3): 146.0; 140.3; 135.5 and 130.0 (C); 22.8 (CH3).

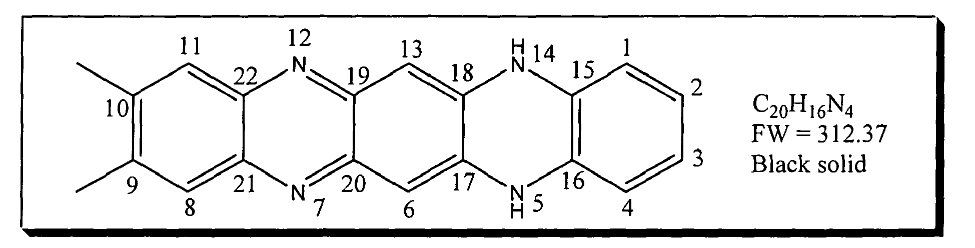

The reaction involves 100 mg (0.416 mmol; 1 eq) of 7,8-dimethyl-2,3-dihydroxy phenazine, 451 mg (4.170 mmol; 10 eq) of *o*-diaminobenzene and 0.4 mL of glacial acetic acid. The reaction residue is taken up in dichloromethane, filtered on sintered glass, rinsed with dichloromethane and then with methanol until obtaining colourless mother liquors. The desired compound is a black solid and is obtained with a yield of 70%.
**¹H-¹³C NMR CPMAS** (10 kHz): 146.6 and 145.0 (C (21) and (22)); 139.4 (C (17), (18), (19) and (20)); 135.8 (C (9) and (10)); 130.5 (C (15) and (16)); 125.2 (CH (8) and (11)); 121.5 (CH (2) and (3)); 113.8 (CH (1) and (4)); 99.7 (CH(6) and (13)); 21.5 and 17.7 (2 CH₃).
**¹H-¹³C NMR CPPI** (C, CH3): 146.6; 145.0; 139.4; 135.8 and 130.5 (C); 21.5 and 17.7 (2 CH₃).
**MS:** [M+H]⁺ = 312.8
**EA:** C = 73.33; H = 5.15; N = 16.64; C₂₀H₁₆N₄.H₂O: C = 72.71; H = 5.49; N = 16.96.

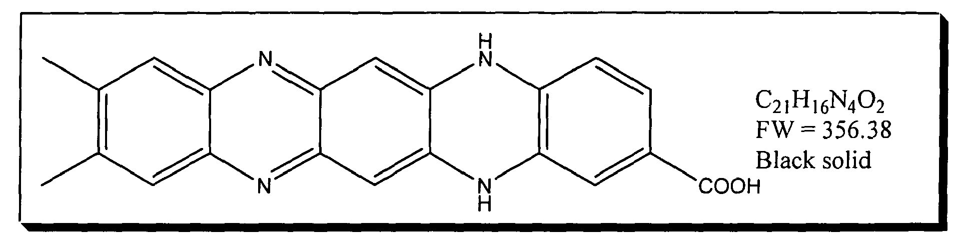

The reaction involves 100 mg (0.390 mmol; 1 eq) of 7-carboxy-2,3-dihydroxy phenazine, 531.6 mg (3.903 mmol; 10 eq) of 4,5-dimethyl-1,2-diamino benzene and 0.4 mL of glacial acetic acid. The reaction residue is taken up in dichloromethane, filtered on sintered glass, rinsed with dichloromethane and then with methanol until obtaining colourless mother liquors. The desired compound is a black solid and is obtained with a yield of 70%.
**¹H NMR** (DMSO *d₆*, ppm): 12.36 (se; 1 H; COOH); 9.85 and 9.62 (2 s; 2*1H; 2 NH); 7.47 (se; 2H; H(8) and (11)); 7.17 (dd; 1H; H(3)); 6.98 (d; 1H; H(1)); 6.46 (d; 1 H; H(4)); 6.38 (s; 1 H; H(6)); 6.31 (s; 1 H; H(13)); 2.35 (s; 6H; 2 CH₃).
**MS:** [M+H]⁺ = 357.8

### PROPERTIES OF THE COMPOUNDS OF THE INVENTION

The conjugated pi-system of the pentacyclic core of the substituted DHTAPs of the invention rearranges depending on the electronic effect of the substituent(s).

The compounds of the invention can be used in photovoltaic or organic solar cells as the method of the invention allows the introduction of grafting groups, such as systems of Graetzel type.

Such non-protonated compounds of the invention have the following absorption properties (in solution): from 450 to 600 nm (ε = 40 000-60 000 L.mol⁻¹.cm⁻¹).

Such non-protonated compounds of the invention have the following absorption properties (in thin film): from 450 to 670 nm.

Such protonated compounds of the invention have the following absorption properties (in solution): from 450 to 700 nm.

The compounds of the invention can be used in organic light emitting diodes (OLED) as the method of the invention allows the introduction of grafting groups, such as hybrid systems.

Such non-protonated compounds of the invention have the following absorption properties in solution: from 450 to 600 nm (ε = 40 000-60 000 L.mol⁻¹.cm⁻¹).

Such non-protonated compounds of the invention have the following fluorescence properties (in solution): from 550 to 700 nm.

Such non-protonated compounds of the invention have the following absorption properties (in solution): from 450 to 700 nm.

Such protonated compounds of the invention have the following fluorescence properties (in solution): from 650 to 750 nm.

## Claims

1. A method for the preparation of compounds having the following formula (I): 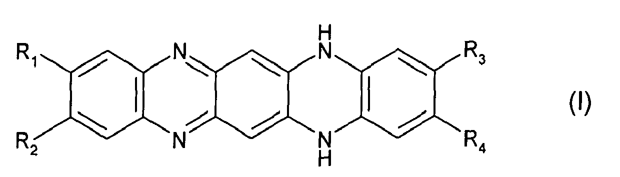 in which:
R₁, R₂, R₃, and R₄ represent, independently from each other, a substituent selected from the group consisting of:
. H
. alkyl group having from 1 to 20, and preferably from 1 to 10, carbon atoms,
. alkenyl group having from 1 to 6 carbon atoms,
. alkynyl group having from 1 to 6 carbon atoms,
. aryl or heteroaryl group having 6 to 30, and preferably from 6 to 10, carbon atoms,
. (C₆-C₁₀)aryl(C₁-C₄)alkyl group,
. acyl group having from 1 to 20 carbon atoms, in particular CO-R group, wherein R is H, an alkyl group, an alkenyl group, an alkynyl group, or an aryl group, as defined above,
. NR'R", wherein R' and R" represent, independently from each other, H, an alkyl group, an alkenyl group, an alkynyl group, or an aryl group, as defined above,
. CONR'R", wherein R' and R" are as defined above,
. NHCOR, wherein R is as defined above,
. COOR, wherein R is as defined above,
. OCOR, wherein R is as defined above,
. OR, wherein R is as defined above,
. SR, wherein R is as defined above,
. SO₂R, wherein R is as defined above,
. SO₂NR'R", wherein R' and R" are as defined above,
. SO₃H,
. halogen such as Cl, F, Br or I,
. CF₃,
. CN, and
. nitro,
or salts thereof,
said method comprising the reaction of a compound of formula (II) 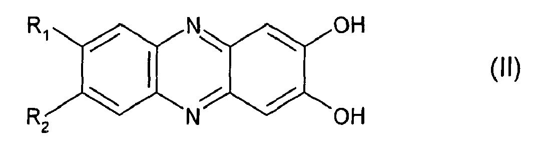 R₁ and R₂ being as defined above,
with a compound having the following formula (III): 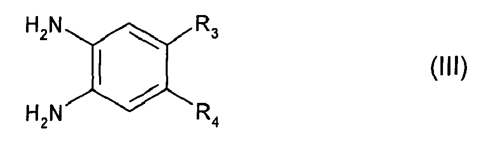 R₃ and R₄ being as defined above,
in the presence of a catalytic amount of an acid selected from the group consisting of: acetic acid, trifluoroacetic acid, and diluted hydrochloric acid.

2. The method of claim 1, wherein the compound of formula (II) is obtained by the reaction of 2,5-dihydroxy-1,4-benzoquinone with a compound having the following formula (IV): 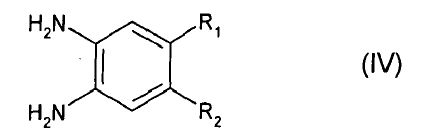 R₁ and R₂ being as defined in claim 1,
said reaction being carried out in water or ethanol, or mixtures thereof.

3. A method for the preparation of compounds having the following formula (I): 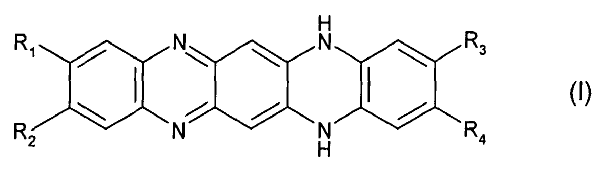 in which:
R₁, R₂, R₃, and R₄ represent, independently from each other, a substituent selected from the group consisting of:
. H
. alkyl group having from 1 to 20, and preferably from 1 to 10, carbon atoms,
. alkenyl group having from 1 to 6 carbon atoms,
. alkynyl group having from 1 to 6 carbon atoms,
. aryl or heteroaryl group having 6 to 30, and preferably from 6 to 10, carbon atoms,
. (C₆-C₁₀)aryl(C₁-C₄)alkyl group,
. acyl group having from 1 to 20 carbon atoms, in particular CO-R group, wherein R is H, an alkyl group, an alkenyl group, an alkynyl group, or an aryl group, as defined above,
. NR'R", wherein R' and R" represent, independently from each other, H, an alkyl group, an alkenyl group, an alkynyl group, or an aryl group, as defined above,
. CONR'R", wherein R' and R" are as defined above,
. NHCOR, wherein R is as defined above,
. COOR, wherein R is as defined above,
. OCOR, wherein R is as defined above,
. OR, wherein R is as defined above,
. SR, wherein R is as defined above,
. SO₂R, wherein R is as defined above,
. SO₂NR'R", wherein R' and R" are as defined above,
. SO₃H,
. halogen such as Cl, F, Br or I,
. CF₃,
. CN, and
. nitro,
or salts thereof,
said method comprising:
a) the reaction of 2,5-dihydroxy-1,4-benzoquinone with a compound having the following formula (IV): 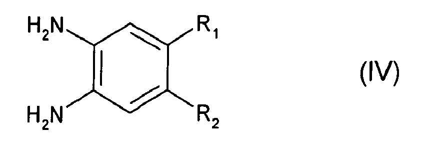 R₁ and R₂ being as defined in claim 1,
said reaction being carried out in water or ethanol, or mixtures thereof,
to obtain a compound of formula (II) as follows: 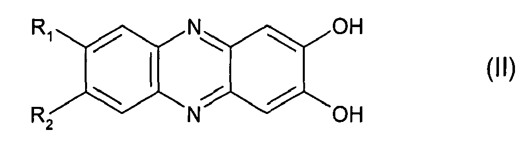 R₁ and R₂ being as defined above,
said compound of formula (II) being then isolated,
b) and the reaction of said compound of formula (II) with a compound having the following formula (III): 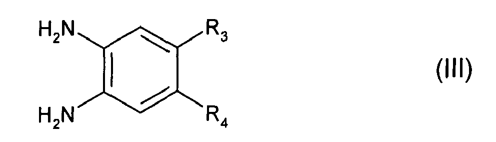 R₃ and R₄ being as defined above,
in the presence of a catalytic amount of an acid selected from the group
consisting of: acetic acid, trifluoroacetic acid, and diluted hydrochloric acid, to obtain said compound of formula (I).

4. A compound having the following formula (I): 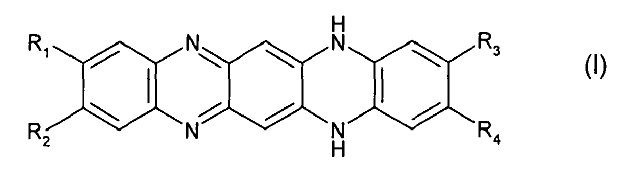 in which:
R₁, R₂, R₃, and R₄ represent, independently from each other, a substituent selected from the group consisting of:
. H
. alkyl group having from 1 to 20, and preferably from 1 to 10, carbon atoms,
. alkenyl group having from 1 to 6 carbon atoms,
. alkynyl group having from 1 to 6 carbon atoms,
. aryl or heteroaryl group having 6 to 30, and preferably from 6 to 10, carbon atoms,
. (C₆-C₁₀)aryl(C₁-C₄)alkyl group,
. acyl group having from 1 to 20 carbon atoms, in particular CO-R group, wherein R is H, an alkyl group, an alkenyl group, an alkynyl group, or an aryl group, as defined above,
. NR'R", wherein R' and R" represent, independently from each other, H, an alkyl group, an alkenyl grou, an alkynyl group, or an aryl group, as defined above,
. CONR'R", wherein R' and R" are as defined above,
. NHCOR, wherein R is as defined above,
. COOR, wherein R is as defined above,
. OCOR, wherein R is as defined above,
. OR, wherein R is as defined above,
. SR, wherein R is as defined above,
. SO₂R, wherein R is as defined above,
. SO₂NR'R", wherein R' and R" are as defined above,
. SO₃H,
. halogen such as Cl, F, Br or I,
. CF₃,
. CN, and
. nitro,
or salts thereof.

5. The compound of claim 4, wherein R₁ and R₂ are H.

6. The compound of any of claims 4 or 5, wherein R₁ and R₂ are alkyl groups such as defined in claim 4, and in particular methyl groups.

7. The compound of any of claims 4 to 6, wherein R₄ is COOR, R being as defined in claim 4, and being preferably H.

8. The compound of any of claims 4 to 6, wherein R₄ is a halogen atom, in particular Cl.

9. An intermediate compound having the following formula (II): 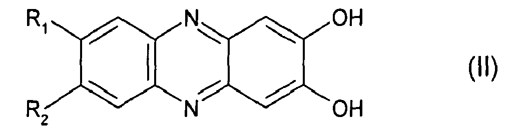 in which:
R₁, and R₂ represent, independently from each other, a substituent selected from the group consisting of:
. H
. alkyl group having from 1 to 20, and preferably from 1 to 10, carbon atoms,
. alkenyl group having from 1 to 6 carbon atoms,
. alkynyl group having from 1 to 6 carbon atoms,
. aryl or heteroaryl group having 6 to 30, and preferably from 6 to 10, carbon atoms,
. (C₆-C₁₀)aryl(C₁-C₄)alkyl group,
. acyl group having from 1 to 20 carbon atoms, in particular CO-R group, wherein R is H, an alkyl group, an alkenyl group, an alkynyl group, or an aryl group, as defined above,
. NR'R", wherein R' and R" represent, independently from each other, H, an alkyl group, an alkenyl group, an alkynyl group, or an aryl group, as defined above,
. CONR'R", wherein R' and R" are as defined above,
. NHCOR, wherein R is as defined above,
. COOR, wherein R is as defined above,
. OCOR, wherein R is as defined above,
. OR, wherein R is as defined above,
. SR, wherein R is as defined above,
. SO₂R, wherein R is as defined above,
. SO₂NR'R", wherein R' and R" are as defined above,
. SO₃H,
. halogen such as Cl, F, Br or I,
. CF₃,
. CN, and
. nitro,
with the proviso that when R₁ is H, then R₂ is not H, and that when R₂ is H, then R₁ is not H.

10. The use of a compound of any of claims 4 to 8, as a chromophore or as a semi-conductive material.

11. The use of a compound of claim 4, wherein at least one of R₁, R₂, R₃, and R₄ groups is COOH, as a light-emitting molecule.

12. The use of a compound of any of claims 4 to 8, as a dye or pigment.

13. A cosmetic composition comprising a compound of any of claims 4 to 8, in association with a cosmetically acceptable vehicle.

14. A method for the cosmetic treatment, comprising the administration of a compound of any of claims 4 to 8, or of the cosmetic composition of claim 13.
